# DEMANDE DE BREVET EUROPEEN

(11) **EP 2 377 561 A1**
(43) Date de publication de la demande: **19.10.2011**
(21) Numéro de dépôt: 11161944.1
(22) Date de dépôt: 11.04.2011
(51) Int. Cl.: A61L 9/12

(54) **Dispositif de diffusion d'une substance volatile**

(30) Priorité: 16.04.2010 FR 1052920
(71) Demandeur: Eurvest, 1420 Braine L'Alleud (BE)
(72) Inventeur: Luciani, Alain, 13390 Auriol (FR); Trimardeau, Gilles, 1420 Braine L'Alleud (BE); Anrys, Séverine, 1420 Braine L'Alleud (BE); Bonnin, Anne Valérie, 1420 Braine L'Alleud (BE); Gasser, Christophe, 1420 Braine L'Alleud (BE); Calvet, Clémentine, 1420 Braine L'Alleud (BE)
(74) Mandataire: Hirsch & Associés

(57) **Abrégé**

Un dispositif de diffusion de substance volatile comprenant un support souple (1) en conformé avec au moins un réceptacle (2) contenant la substance volatile à diffuser et avec au moins une languette (3), un opercule recouvrant au moins le réceptacle (2). La languette est adaptée à être rabattue par-dessus le réceptacle.

Le dispositif selon l'invention peut être fabriqué en ligne sans étape d'assemblage. La languette souple permet de protéger la surface de diffusion et de modeler le dispositif selon différentes formes.

## Description

L'invention concerne un dispositif de diffusion d'une substance volatile et un procédé de fabrication d'un tel dispositif.

Un dispositif de diffusion de substance volatile comprend typiquement un support dans lequel est accueilli un réservoir contenant la substance volatile à diffuser. La substance volatile à diffuser peut être un parfum éventuellement associé à une formulation assainissante et/ou à une formulation contenant des actifs insecticides, comme par exemple de l'extrait de margosa, des pyrèthres naturels, de l'huile de lavandin ou du géraniol pour une action antimite. Un tel dispositif est généralement conçu pour être placé dans une armoire, un tiroir, une penderie ou simplement dans une pièce ou un véhicule.

Le document US-A-5 961 043 décrit un dispositif de diffusion de substance volatile comprenant un support rigide présentant une ouverture pour recevoir un réservoir contenant la substance à diffuser. Le support comprend deux panneaux qui peuvent être rejoints en formant une base grâce à laquelle le dispositif peut être posé en configuration de tente sans avoir à être accroché.

Le dispositif décrit dans ce document est relativement complexe. Un support en carton ou en plastique rigide est formé avec une ouverture destinée à recevoir le réservoir de la substance à diffuser. Le réservoir est fabriqué et rempli séparément puis agencé dans l'ouverture du support. Deux lignes de fabrication doivent donc être prévues avec une étape d'assemblage du réservoir dans le support.

En outre, le dispositif décrit dans ce document est rigide et par conséquent les possibilités de mise en forme sont limitées.

Par ailleurs, la formulation contenant la substance volatile à diffuser peut également comprendre un colorant. Un contact avec la surface de diffusion peut alors entrainer un transfert du colorant, par exemple sur du linge lorsque le dispositif est utilisé dans une penderie ou un tiroir.

Il existe donc un besoin pour un dispositif de diffusion de substance volatile qui soit simple de fabrication, qui autorise diverses mises en forme et qui permette de protéger la surface de diffusion.

A cet effet l'invention propose un dispositif de diffusion de substance volatile comprenant un support souple formé avec au moins une zone définissant un réceptacle de la substance à diffuser et au moins une zone définissant une languette. La souplesse du support est assurée à la fois par le choix du matériau utilisé pour thermoformer le support et par la faible épaisseur du matériau. Le support du dispositif selon l'invention présente ainsi une texture fine et souple qui permet de plier et modeler le support à volonté.

Le dispositif selon l'invention ne présente donc pas de réservoir séparé du support qui doit être assemblé avec ce dernier. Le réservoir est intégré au support, i.e. le réceptacle fait partie du support. Le réservoir est donc fabriqué avec le support et l'étape d'assemblage est éliminée. La languette est également intégrée au support, i.e. fait partie du support. La languette est souple et peut être rabattue par-dessus le réceptacle afin de protéger la surface de diffusion et éviter tout risque de transfert de colorant.

Plus particulièrement, l'invention propose un dispositif de diffusion de substance volatile comprenant:
- un support souple conformé avec au moins une zone définissant un réceptacle contenant la substance volatile à diffuser et avec au moins une zone définissant une languette;
- un opercule recouvrant au moins une ouverture du réceptacle,

la languette étant adaptée à être rabattue sans pliure par-dessus le réceptacle.

Selon les modes de réalisation, le dispositif de diffusion de substance volatile selon l'invention peut comprendre une ou plusieurs des caractéristiques suivantes :
- la languette présente un moyen d'accroche adapté à coopérer avec un moyen d'accroche complémentaire du support de manière à maintenir la languette rabattue par-dessus le réceptacle ;
- le moyen d'accroche de la languette est une première encoche et le moyen d'accroche complémentaire du support est une deuxième encoche ;
- le moyen d'accroche de la languette est un onglet et le moyen d'accroche complémentaire du support est une fente ;
- le moyen d'accroche de la languette est multiple et/ou le moyen d'accroche complémentaire du support est multiple ;
- le support est conformé avec en outre un crochet ;
- le support est conformé avec au moins deux languettes ;
- l'opercule recouvre sensiblement tout le support ;
- le dispositif comprend en outre une couche barrière à la diffusion de la substance volatile, ladite couche barrière étant pelable ;
- le support est en thermoplastique ;
- le support est constitué d'une couche de polymère d'une épaisseur comprise entre 300 µm et 600 µm ;
- l'opercule est constitué d'une couche de polymère d'une épaisseur comprise entre 30 µm et 70 µm ;
- le réceptacle du support présente une forme sensiblement demi-sphérique ;

L'invention concerne aussi un procédé de fabrication d'un dispositif de diffusion de substance volatile selon l'invention, comprenant les étapes consistant à :
- thermoformer un support selon une géométrie tridimensionnelle présentant au moins un réceptacle, le support étant constitué d'une couche de polymère d'une épaisseur comprise entre 300 µm et 600 µm ;
- remplir le réceptacle avec une formulation contenant la substance volatile à diffuser ;
- sceller le réceptacle avec un opercule ;
- découper le support selon une géométrie présentant au moins une languette, l'ensemble desdites étapes étant réalisées en ligne continue.

D'autres caractéristiques et avantages de l'invention apparaîtront à la lecture de la description détaillée qui suit. Cette description est donnée à titre d'exemple uniquement, et en référence aux figures annexées qui montrent :
- figure 1, une vue schématique en perspective de dessus d'un dispositif de diffusion de substance volatile selon l'invention, selon un premier mode de réalisation ;
- figure 2, une vue schématique en coupe AA du dispositif de la figure 1 ;
- figure 3, une vue schématique en perspective de dessus d'un dispositif de diffusion de substance volatile selon l'invention, selon un deuxième mode de réalisation ;
- figure 4, une vue schématique en perspective de dessus d'un dispositif de diffusion de substance volatile selon l'invention, selon un troisième mode de réalisation ;
- figure 5, une vue schématique en perspective de dessus d'un dispositif de diffusion de substance volatile selon l'invention, selon un quatrième mode de réalisation ;
- figure 6, une vue schématique en perspective du dispositif de la figure 4 avec les languettes accrochées ;
- figure 7, une vue schématique en perspective du dispositif de la figure 1 avec la languette accrochée.

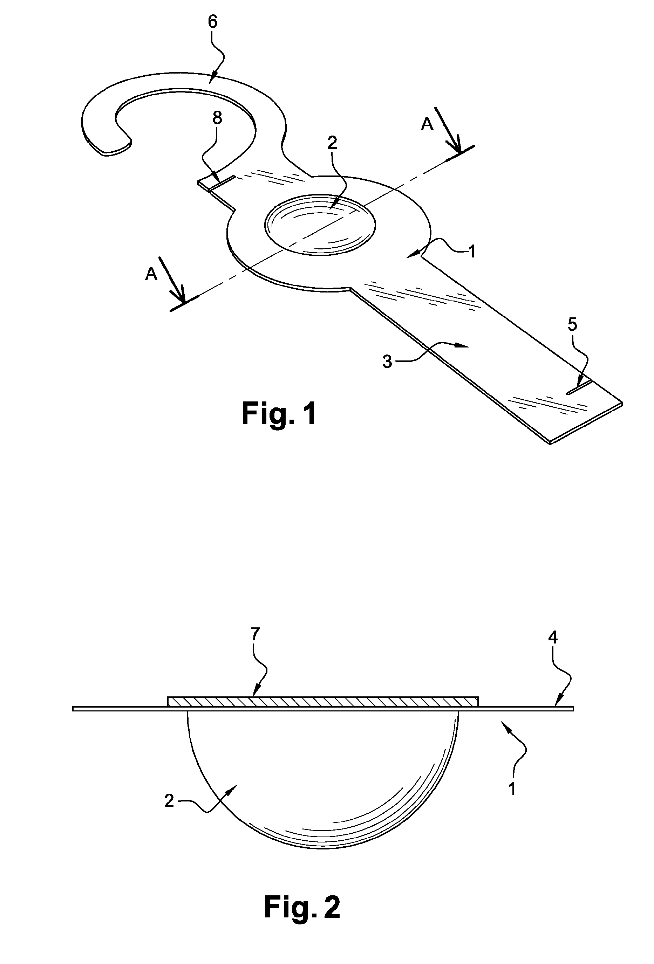

Le dispositif de diffusion selon l'invention va être décrit en référence à la figure 1 qui montre un premier mode de réalisation.

La figure 1 montre un support 1 présentant une zone dans laquelle est formé un réceptacle 2 destiné à contenir la substance volatile à diffuser. Le support 1 présente aussi une zone dans laquelle est formée une languette 3 et une zone dans laquelle est formée un crochet 6.

Le support 1 est souple. Il peut être composé d'un matériau thermoplastique adapté au thermoformage. Par exemple, le support 1 peut être constitué d'une couche de polymère d'une épaisseur comprise entre 300 µm et 600 µm. Cette couche de polymère est thermoformée pour intégrer directement au support 1 le réceptacle 2 et la languette 3, ainsi que le crochet 6 dans le mode de réalisation de la figure 1. La couche polymère constituant le support 1 peut être du polyéthylène, du polypropylène, du polyéthylène téréphtalate, ou un mélange de ceux-ci. La couche polymère constituant le support 1 peut aussi être du biopolymère tel que de l'acétate de cellulose, du nylon 11, du polyhydroxyalkanoate, de l'acide polylactique ou un mélange de ceux-ci.

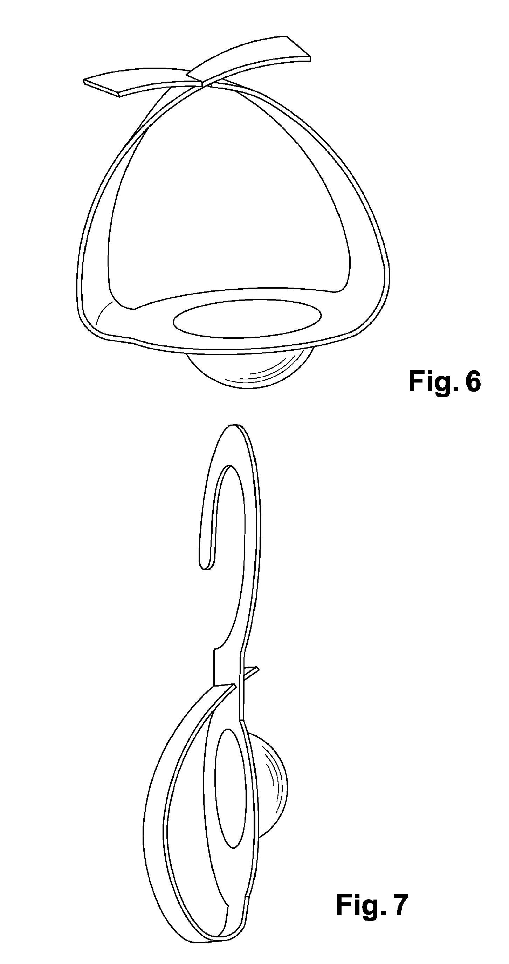

La figure 1 montre également une encoche 5 ménagée sur la languette 3 et une encoche 8 ménagée dans le support 1. Ces encoches 5, 8 sont destinées à coopérer pour permettre un accrochage de la languette 3 en position rabattue par-dessus le réceptacle 2, comme cela est illustré sur la figure 7.

Comme exposé plus haut, la languette 3 fait partie du support 1 ; elle est donc souple. Cette souplesse est conférée d'une part par la nature du matériau utilisé pour constituer le support et d'autre part par l'épaisseur du support. La languette peut ainsi être rabattue par-dessus la surface de diffusion du dispositif pour modeler le dispositif selon différentes formes : forme aplatie de la figure 1 ou forme arrondie de la figure 7. Notamment, la languette 3 peut être rabattue par-dessus le réceptacle 2 sans former une ligne de pliure et sans suivre une ligne de pliure prédéfinie. Plusieurs encoches 5, 8 peuvent être prévues (non illustrées) sur le support 1 et/ou sur la languette 3 afin de permettre une forme plus ou moins ovale ou plus ou moins sphérique du dispositif. Ces formes plus ou moins ovales ou sphériques peuvent être obtenues avec un même dispositif parce que la souplesse du support 1 permet de rabattre la languette 2 sans suivre une ligne de pliure prédéfinie.

La figure 2 montre une vue en coupe AA du dispositif de la figure 1. On suppose que le réceptacle 2 du dispositif est rempli d'une formulation contenant la substance à diffuser. Un opercule 4 est déposé sur le support 1 afin de recouvrir au moins la zone de diffusion du réceptacle 2. L'opercule 4 peut tout à fait recouvrir l'ensemble du support 1. L'opercule 4 peut être appliqué à chaud sur le support 1 thermoformé juste après remplissage du réceptacle 2 par la formulation contenant la substance volatile à diffuser.

L'opercule 4 autorise une diffusion de la substance volatile, c'est-à-dire le passage de molécules de parfum et/ou de molécules de la substance active insecticide. L'opercule 4 peut être constitué d'une couche de polymère d'une épaisseur comprise entre 30 µm et 70 µm. La couche polymère constituant l'opercule 4 peut être du polyéthylène, du polypropylène, du polyéthylène téréphtalate, ou un mélange de ceux-ci. La couche polymère constituant l'opercule 4 peut aussi être du biopolymère tel que de l'acétate de cellulose, du nylon 11, du polyhydroxyalkanoate, de l'acide polylactique ou un mélange de ceux-ci.

La figure 2 montre également une couche barrière 7 qui prévient la diffusion de la substance volatile avant utilisation du dispositif. Une telle couche barrière 7 peut être en aluminium ou en polymère d'une épaisseur telle que la diffusion est empêchée. La couche barrière 7 est de préférence pelable, c'est-à-dire qu'elle peut être retirée manuellement par traction sans effort notable et sans endommager l'opercule 4. La couche barrière 7 recouvre au moins la zone de diffusion du réceptacle 2 mais peut tout à fait recouvrir l'ensemble du support 1 par-dessus la couche d'opercule 4.

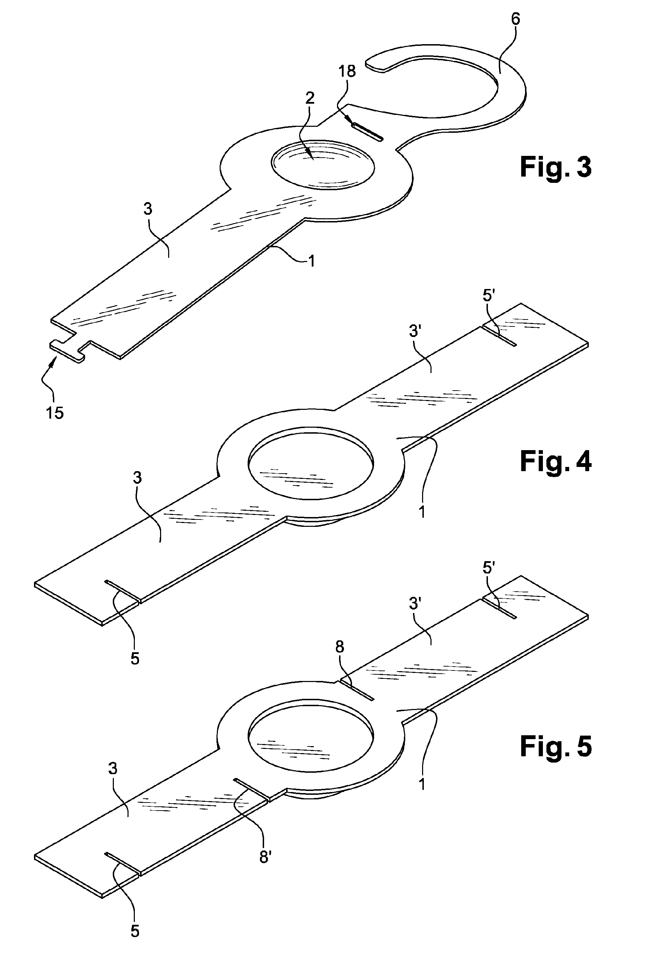

Le dispositif de diffusion selon l'invention va maintenant être décrit en référence à la figure 3 qui montre un deuxième mode de réalisation. Les éléments communs au premier mode de réalisation décrit ci-dessus portent les mêmes numéros de référence et ne sont pas décrits à nouveau.

Le mode de réalisation de la figure 3 diffère du mode de réalisation de la figure 1 par les moyens d'accroche utilisés pour maintenir la languette 3 dans une position rabattue par-dessus le réceptacle 2. Selon ce deuxième mode de réalisation, la languette 3 présente un onglet 15 et le support présente une fente 18 adaptée à recevoir l'onglet 15 pour permettre un accrochage de la languette 3 en position rabattue par-dessus le réceptacle 2. Plusieurs fentes 18 peuvent être prévues sur le support 1 (non illustrées) afin de permettre une forme plus ou moins ovale ou plus ou moins sphérique du dispositif.

Le dispositif de diffusion selon l'invention va encore être décrit en référence aux figures 4 et 5 qui montrent respectivement un troisième et un quatrième mode de réalisation. Les éléments communs au premier mode de réalisation décrit ci-dessus portent les mêmes numéros de référence et ne sont pas décrits à nouveau.

Le mode de réalisation de la figure 4 diffère du mode de réalisation de la figure 1 par la présence d'une deuxième languette 3'. Sur la figure 4, chaque languette 3, 3' présente une encoche 5, 5', ces encoches coopérant pour maintenir chaque languette rabattue par-dessus le réceptacle tel que cela est illustré sur la figure 6. Chaque encoche constitue à la fois le moyen d'accroche de la languette sur laquelle elle est positionnée et le moyen d'accroche complémentaire du support pour la languette opposée.

Bien que non illustré, les modes de réalisation des figures 3 et 4 peuvent être combinés, c'est-à-dire que les moyens d'accroche décrits en référence à la figure 3 peuvent être adaptés à un dispositif de diffusion comprenant deux languettes. Une première languette 3 pourrait présenter un ergot 15 comme illustré figure 3 et la deuxième languette 3' pourrait présenter une fente 18 comme illustré figure 3.

Le mode de réalisation de la figure 5 diffère du mode de réalisation de la figure 4 par la présence d'encoches 8, 8' sur le support 1 en plus des encoches 5, 5' de chaque languette 3, 3'. Chaque languette 3, 3' peut alors être rabattue par-dessus le réceptacle 2 et maintenue dans cette position par son encoche 5, 5' insérée dans une encoche 8, 8' ménagée dans le support à proximité du réceptacle 2. On obtiendrait ainsi un croisement et une superposition des languettes 3, 3' au dessus du réceptacle. Les positions des encoches 5, 5', 8, 8' déterminent l'aspect plus ou moins arrondi du dispositif. Par exemple, plusieurs encoches peuvent être prévues à quelques millimètres d'intervalle sur les languettes 3, 3' et/ou le support 1 pour ajuster le dispositif à la forme souhaitée.

Bien que cela ne soit pas représenté, on pourrait également envisager un dispositif comprenant un support avec trois ou quatre languettes ou davantage, chaque languette étant adaptée à être rabattue par-dessus le réceptacle. Par exemple, le mode de réalisation de la figure 5 peut être modifié avec une configuration en étoile des languettes et un positionnement adéquat des encoches sur le support et les languettes.

De plus, bien que cela ne soit pas représenté, on pourrait également envisager un dispositif comprenant un support avec au moins deux languettes, une première languette étant rabattue par-dessus l'opercule recouvrant le réceptacle et une deuxième languette étant rabattue par-dessus l'arrière du réceptacle.

Quel que soit le mode de réalisation, le dispositif de diffusion de substance volatile selon l'invention peut être modelé selon différentes formes et permet une protection de la surface de diffusion. Lorsque la languette est rabattue par-dessus le réceptacle, le dispositif présente une forme arrondie (plus ou moins ovoïdale ou plus ou moins sphérique). Un tel aspect arrondi du dispositif peut être obtenu grâce à la souplesse du support 1 qui permet de rabattre la ou les languettes sans suivre une ou des lignes de pliure. Afin d'accentuer et de compléter cette forme arrondie, le réceptacle peut présenter une forme sensiblement demi-sphérique. Cette forme du réceptacle peut être obtenue lors du thermoformage du support.

Le dispositif de diffusion de substance volatile selon l'invention peut être fabriqué comme suit.

Une couche de polymère d'une épaisseur comprise entre 300 µm et 600 µm est thermoformée selon une géométrie tridimensionnelle pour former un support présentant au moins un réceptacle.

Le réceptacle est rempli avec une formulation contenant la substance volatile à diffuser puis scellé avec un opercule. L'opercule peut être scellé à chaud sur toute la surface du support.

Le support est alors découpé selon une géométrie présentant au moins une languette ainsi que des encoches et/ou fentes et ergots. La découpe peut être effectuée à l'emporte-pièce.

L'ensemble de ces étapes peut être réalisé en ligne continue au moyen d'une technologie connue sous l'acronyme FFS (Form Fill and Seal en anglais pour former, remplir et sceller).

Bien entendu, la présente invention n'est pas limitée aux modes de réalisation décrits à titre d'exemple.

Notamment, la forme et les dimensions du support 1 peuvent être sujettes à modifications et d'autres matériaux que ceux décrits ci-dessus peuvent être utilisés pour former le support tant qu'ils permettent au support de conserver un caractère de souplesse permettant de rabattre la languette par dessus du réceptacle. Le réceptacle 2 peut être rempli avec toute formulation connue ou à venir contenant une ou des substances actives à diffuser.

En outre, d'autres moyens d'accroches que ceux décrit ci-dessus peuvent être envisagés tels que des pastilles adhésives appliquées au support et à la languette par exemple.

## Revendications

1. Dispositif de diffusion de substance volatile comprenant:
- un support souple (1) conformé avec au moins une zone définissant un réceptacle (2) contenant la substance volatile à diffuser et avec au moins une zone définissant une languette (3);
- un opercule (4) recouvrant au moins une ouverture du réceptacle (2),
la languette (3) étant adaptée à être rabattue sans pliure par-dessus le réceptacle (2).

2. Dispositif de diffusion de substance volatile selon la revendication 1 dans lequel la languette (3) présente un moyen d'accroche (5, 5', 15) adapté à coopérer avec un moyen d'accroche complémentaire (8, 8', 18) du support (1) de manière à maintenir la languette (3) rabattue par-dessus le réceptacle (2).

3. Dispositif de diffusion de substance volatile selon la revendication 2 dans lequel le moyen d'accroche de la languette est une première encoche (5, 5') et le moyen d'accroche complémentaire du support est une deuxième encoche (8, 8').

4. Dispositif de diffusion de substance volatile selon la revendication 2 dans lequel le moyen d'accroche de la languette est un onglet (15) et le moyen d'accroche complémentaire du support est une fente (18).

5. Dispositif de diffusion de substance volatile selon l'une quelconque des revendications 2 à 4 dans lequel le moyen d'accroche de la languette est multiple et/ou le moyen d'accroche complémentaire du support est multiple.

6. Dispositif de diffusion de substance volatile selon l'une quelconque des revendications précédentes dans lequel le support (1) est conformé avec en outre un crochet (6).

7. Dispositif de diffusion de substance volatile selon l'une quelconque des revendications précédentes dans lequel le support (1) est conformé avec au moins deux languettes (3, 3').

8. Dispositif de diffusion de substance volatile selon l'une quelconque des revendications précédentes dans lequel l'opercule (4) recouvre sensiblement tout le support (1).

9. Dispositif de diffusion de substance volatile selon l'une quelconque des revendications précédentes comprenant en outre une couche barrière (7) à la diffusion de la substance volatile, ladite couche barrière étant pelable.

10. Dispositif de diffusion de substance volatile selon l'une quelconque des revendications précédentes dans lequel le support (1) est en thermoplastique.

11. Dispositif de diffusion de substance volatile la revendication 10 dans lequel le support (1) est constitué d'une couche de polymère d'une épaisseur comprise entre 300 µm et 600 µm.

12. Dispositif de diffusion de substance volatile selon l'une quelconque des revendications précédentes dans lequel l'opercule (4) est constitué d'une couche de polymère d'une épaisseur comprise entre 30 µm et 70 µm.

13. Dispositif de diffusion de substance volatile selon l'une quelconque des revendications précédentes dans lequel le réceptacle (2) du support (1) présente une forme sensiblement demi-sphérique.

14. Procédé de fabrication d'un dispositif de diffusion de substance volatile selon l'une des revendications 1 à 13, comprenant les étapes consistant à :
- thermoformer un support (1) selon une géométrie tridimensionnelle présentant au moins un réceptacle (2), le support (1) étant constitué d'une couche de polymère d'une épaisseur comprise entre 300 µm et 600 µm ;
- remplir le réceptacle (2) avec une formulation contenant la substance volatile à diffuser ;
- sceller le réceptacle (2) avec un opercule (4) ;
- découper le support (1) selon une géométrie présentant au moins une languette (3),
l'ensemble desdites étapes étant réalisées en ligne continue.
